(19)

Europäisches
Patentamt
European
Patent Office
Office européen
des brevets

(11) **EP 4 446 346 A1**

(12) **EUROPEAN PATENT APPLICATION**
published in accordance with Art. 153(4) EPC

(43) Date of publication:
**16.10.2024 Bulletin 2024/42**

(21) Application number: **22904306.2**

(22) Date of filing: **08.12.2022**

(51) International Patent Classification (IPC):
*C07K 17/12* (2006.01)     *C08J 9/28* (2006.01)
*C12N 5/0789* (2010.01)

(52) Cooperative Patent Classification (CPC):
**C07K 17/12; C08J 9/28; C12N 5/06**

(86) International application number:
**PCT/JP2022/045340**

(87) International publication number:
**WO 2023/106379 (15.06.2023 Gazette 2023/24)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **08.12.2021 JP 2021199524**

(71) Applicants:
• **The University of Tokyo**
**Bunkyo-ku, Tokyo 113-8654 (JP)**
• **Asahi Kasei Kabushiki Kaisha**
**Tokyo 1000006 (JP)**

(72) Inventors:
• **HARA, Yuichi**
**Tokyo 100-0006 (JP)**

• **HARANO, Kodai**
**Tokyo 100-0006 (JP)**
• **SANO, Ayae**
**Tokyo 100-0006 (JP)**
• **ITO, Taichi**
**Tokyo 113-8654 (JP)**
• **OKI, Yuichiro**
**Tokyo 113-8654 (JP)**
• **INAGAKI, Natsuko**
**Tokyo 113-8654 (JP)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(54) **CELLULOSE POROUS PARTICLES, AND MICROCARRIER FOR CULTURE USE WHICH COMPRISES SAME**

(57)     Provided are: cellulose porous particles which have large surface areas on which cells can be attached, and on which cells can be adhered easily, and which rarely undergo the detachment of cells when the cells are cultured at a high density; and a microcarrier for culture use, which comprises the cellulose porous particles.

The cellulose porous particles according to the present invention are composed of cellulose, in which the cellulose constituting the cellulose porous particles is modified by a cationic substituent and the cellulose constituting the cellulose porous particles has a polypeptide bound thereto.

EP 4 446 346 A1

**Description**

FIELD

The present invention relates to cellulose porous particles to be used for cell culturing, and to a culturing microcarrier comprising the cellulose porous particles.

BACKGROUND

Efficient and large-scale culturing of cells, tissues and microorganisms is necessary in fields such as production of vaccines and antibody drugs, and regenerative medicine.

Methods using microcarriers are currently in wide use as mass cell culturing techniques for culturing of adhesion-dependent cells because they widen the culture surface area with small volumes, while simultaneously making it possible to maintain high cell density per culture volume, as compared to monolayer culture methods which use flasks or roller bottles with low culture surface area/volume ratios.

Cytodex 1 (product of Cytiva) particles having about 1.5 mmol/g charge capacity, as dextran particles modified with cationic diethylaminoethyl groups, are widely used as microcarriers, but culturing of cells takes place only on the surfaces since the particles do not have a porous structure, and this has limited their proliferation potency. When agitation culture is carried out in a reactor, generated shear stress has been a problem because it causes shedding of cells and significantly lowers culturing efficiency.

Porous microcarriers with large surface areas have been developed with an aim to solve these problems. PTL 1 describes culturing using cellulose porous particles with the feature of mutually communicating pores having pore sizes larger than about 2 μm. Culturing using such porous particles allows cells to be cultured efficiently due to their large surface area, while also helping to avoid the effects of shear stress during agitation culture since the cells are situated in the particle interiors.

[CITATION LIST]

[PATENT LITERATURE]

[PTL 1] Japanese Unexamined Patent Publication HEI No. 2-208331
[PTL 2] Japanese Unexamined Patent Publication HEI No. 5-252941

SUMMARY

[TECHNICAL PROBLEM]

With a culturing microcarrier using cellulose porous particles as described in PTL 1, at the initial stage of culturing where substrate adhesion between the microcarrier and cells is still undeveloped with introduction of charge onto the carrier surfaces, the cells are rapidly adsorbed onto the surfaces of the porous microcarrier. However, shear stress resistance may be weak depending on the type of cell, creating a problem in that the cells may be shed during initial culturing, thus lowering their proliferative ability. When the proliferative ability is low during initial culturing, various means have been employed for more prolonged culturing to obtain fixed amounts of the cells or substances produced by the cells, but for some types of cells, such prolonged culturing may lower the activity of the cells or their proteins. While methods exist for further increasing surface charge to increase the adsorptive strength and help prevent shedding, the surface charge may become too high depending on the type of cell, which can result in growth inhibition, and therefore a method is needed to inhibit shedding while limiting charge.

The use of cell adhesion factors as means for promoting adhesion of cells onto particle surfaces as a measure against such problems is known. Cell adhesion factors are proteins having the property of specifically adhering to cell surface receptors that are thought to regulate cell adhesion. For example, PTL 2 discloses a porous network structure coated with a peptide that includes the amino acid sequence ArgGlyAsp (RGD), as a cell adhesion factor. However, since the substrate described in PTL 2 is spherical with a large diameter, it requires large stirring force to suspend and evenly disperse the carrier throughout the medium during agitation culture. High shear stress is therefore produced, resulting in shedding of cells disposed on the surface before the original adhesive effect of the adhesion factor is exhibited. Furthermore since polyethyleneimine which has high charge density is used as the cationic substituent, it is difficult to introduce a suitable charge, resulting in growth inhibition for some types of cells. In addition, while polyethyleneimine is also used as a spacer for peptide binding, this polymer has a branched structure which tends to create local imbalance between charge amount and peptide modification, making it difficult for the peptides to be oriented in the proper direction and thereby lowering cell adhesion. With the method of PTL 2, therefore, certain cell types or culturing conditions have a tendency to result in shedding, making it impossible to accomplish efficient cell growth.

In light of this situation of the prior art, the problem to be solved by the invention is to provide cellulose porous particles with excellent cell adhesion and with low cell shedding even when cultured at high density, and allowing high efficiency growth of the cells, as well as a culturing microcarrier comprising them.

[SOLUTION TO PROBLEM]

As a result of avid research on the problems described above, the present inventors have completed this invention based on the unexpected finding that introducing a cell adhesive peptide into porous cellulose particles modified with a cationic substituent reduces shedding of cells during high density culturing and provides excellent cell proliferative ability, and especially cell proliferative ability at the early stage of culturing.

Specifically, the present invention is as follows.

[1] Cellulose porous particles composed of cellulose, wherein the cellulose composing the cellulose porous particles is modified with a cationic substituent, and the cellulose composing the cellulose porous particles has a polypeptide bonded to the cellulose.

[2] The cellulose porous particles according to [1] above, wherein the charge capacity of the porous particles is 0.05 mmol/g to 5.0 mmol/g.

[3] The cellulose porous particles according to [1] or [2] above, wherein at least a portion of the polypeptide is a cell adhesive peptide.

[4] The cellulose porous particles according to any one of [1] to [3] above, wherein at least a portion of the polypeptide is a peptide having the amino acid sequence ArgGlyAsp (RGD).

[5] The cellulose porous particles according to any one of [1] to [4] above, wherein the loading amount of the polypeptide is 10 nmol/g to 1 mmol/g per 1 g of cell culture carrier (dry cellulose porous particles).

[6] The cellulose porous particles according to any one of [1] to [5] above, wherein the loading amount of the polypeptide is 1.1 $\mu$mol/g to 1 mmol/g per 1 g of cell culture carrier (dry cellulose porous particles).

[7] The cellulose porous particles according to any one of [1] to [6] above, wherein the number of amino acid residues in the polypeptide is 5 to 30 residues.

[8] The cellulose porous particles according to any one of [1] to [7] above wherein the specific surface area of the dry cellulose porous particles is 0.3 $m^2$/g to 4.4 $m^2$/g.

[9] The cellulose porous particles according to any one of [1] to [8] above, wherein cellulose has multiple pores, with a mean pore size of 5 $\mu$m to 100 $\mu$m.

[10] The cellulose porous particles according to [9] above, wherein the pores form a continuous pore structure with adjacent pores mutually communicating by openings in the membranes dividing them.

[11] The cellulose porous particles according to any one of [1] to [10] above, which have a mean particle size of 100 $\mu$m to 1000 $\mu$m.

[12] The cellulose porous particles according to any one of [1] to [11] above, wherein the cationic substituent is at least one selected from the group consisting of primary amino, secondary amino and tertiary amino groups.

[13] The cellulose porous particles according to [12] above, wherein the cationic substituent is a tertiary amino group.

[14] The cellulose porous particles according to any one of [1] to [13] above, wherein the polypeptide is bonded to the cellulose via a spacer, the chain length of the spacer being 5 to 20 atoms.

[15] A culturing microcarrier comprising cellulose porous particles according to any one of [1] to [14] above.

[ADVANTAGEOUS EFFECTS OF INVENTION]

The cellulose porous particles of the invention, when used as a microcarrier serving as a culture carrier, allow cells to be efficiently adsorbed during the initial stage of culturing when substrate adhesion between the cells is still undeveloped, while also reducing shedding of cells from the particles by the effects of shear stress even after high density culturing, and allowing the cells to be highly efficiently grown.

DESCRIPTION OF EMBODIMENTS

The invention will now be explained in detail by embodiments.

One embodiment of the invention is cellulose porous particles composed of cellulose, wherein the cellulose composing the cellulose porous particles is modified with a cationic substituent, and the cellulose composing the cellulose porous particles has a polypeptide bonded to the cellulose.

The type of cellulose composing the cellulose porous particles for this embodiment is not particularly restricted, and any publicly known cellulose such as copper-ammonia regenerated cellulose, viscose regenerated cellulose, cotton, pulp or polynosic may be used, among which copper-ammonia regenerated cellulose and viscose regenerated cellulose are preferred, and copper-ammonia regenerated cellulose is more preferred.

The cellulose density is preferably 1.5 g/cm$^3$, as the density for a material to be used as a microcarrier. Using a substance such as metal having a density of 2.0 or greater requires strong stirring force to cause suspension, which increases shear stress and leads to cell damage, and therefore it is not preferred as a microcarrier for use in agitation culture. A substance with a density of less than 1.0, on the other hand, will rise to the liquid surface and allow the cells to contact with air, making them unusable for agitation culture.

Copper-ammonia regenerated cellulose is highly reactive for functional group modifications due to its low crystallinity, and allows uniform modification to be made using spacers and cationic functional groups necessary for introduction of cell adhesion peptides, at high density and even throughout the interiors of porous particles. Copper-ammonia regenerated cellulose also has the feature of lower zeta-potential compared to common crystalline cellulose. It is therefore unlikely to engage in interaction such as electrostatic adsorption or repulsion with cell adhesion peptides, allowing the peptides to be uniformly introduced into the porous interiors, while due to the three-dimensional arrangement on the microcarrier surfaces, the peptides are also able to contact with cells at a suitable orientation.

The form of the cellulose porous particles of the embodiment is preferably spherical, more preferably true spherical with true sphericity of 0.70 to 1.00 and even more preferably true sphericity of 0.90 to 1.00. The term "sphericity" as used herein is a value representing the shapes of the particles, being determined by swelling the porous particles with water and then removing the excess water, measuring the projected area of the particles in an optical microscope image, and comparing the circumference length of a circle having the same area as the projected area with the actual circumference of the particle as seen in an electron microscope image. Although this measuring method only considers the particle on one plane, using the average value for measurement of 100 or more particles makes it possible to account for variation in different observation directions, allowing the three-dimensional sphericity of the particles to be determined. A value closer to 1.00 indicates a more spherical particle shape, with 1.00 representing total sphericity.

Since a spherical porous microcarrier has no anisotropy for interior liquid permeability, cell adhesive peptides can be uniformly modified throughout the particle interiors, allowing the cells to extend uniformly throughout the particle interiors. It is also possible to obtain buoyancy with lower stirring force compared to other shapes such as cubes, lowering the shear stress and helping to reduce shedding.

The lower limit for the mean particle size of the cellulose porous particles of the embodiment is preferably 100 μm or greater and more preferably 200 μm or greater. A mean particle size of greater than 100 μm will result in more cells being held per microcarrier, thus facilitating separation from the medium. The upper limit for the mean particle size of the cellulose porous particles of the embodiment is preferably 1000 μm or smaller, more preferably 500 μm or smaller and even more preferably 400 μm or smaller. A mean particle size of smaller than 1000 μm will allow nutrients and oxygen to be sufficiently supplied to the cells at the center sections of the microcarrier, resulting in a satisfactory culturing environment. A mean particle size of smaller than 1000 μm will also require less stirring force to suspend and disperse the microcarrier, thus lowering the shear stress. Shedding of cells will thus be less likely to occur, thus being more suitable for high-density culture. The term "mean particle size" as used herein is the average particle diameter measured for at least 20 particles, after removing the excess water once the porous particles have been swelled with water, and setting an appropriate magnification with an optical microscope.

The lower limit for the sizes of the pores of the cellulose porous particles of the embodiment is a mean pore size of preferably 5 μm or greater and more preferably 10 μm or greater. A mean pore size of 5 μm or greater will increase the freedom of movement for cells and culture solution throughout the microcarrier. The upper limit for the mean pore size is preferably 100 μm or smaller, more preferably 80 μm or smaller and even more preferably 50 μm or smaller. A mean pore size of smaller than 100 μm can ensure sufficient surface area for cell growth, for efficient proliferation of the cells. The term "mean pore size" as used herein refers to the average pore size as measured by swelling the porous particles with an ionic liquid and removing the excess ionic liquid, and determining the distance between membranes for 3 or more particles in a 2D image taken with a high-resolution 3D X-ray microscope at a resolution of 0.54 μm/pixel, based on analysis using image analysis software (Image J). The pore size was defined as the size of the maximum circle fitting in the space between porous particles in the 2D image, based on the definition used in the Thickness method.

The lower limit for the surface area of the cellulose porous particles of the embodiment per 1 g of dry weight (specific surface area) is preferably 0.3 m$^2$/g or greater, more preferably 0.6 m$^2$/g or greater and even more preferably 0.8 m$^2$/g or greater. The surface area necessary for cell culturing is preferably 0.3 m$^2$/g or greater from the viewpoint of efficient proliferation of the cells. The upper limit for the specific surface area is preferably 4.4 m$^2$/g or lower, more preferably 3.3 m$^2$/g or lower, even more preferably 2.0 m$^2$/g or lower and most preferably 1.7 m$^2$/g or lower. The specific surface area is preferably 4.4 m$^2$/g or lower from the viewpoint of not lowering the charge per unit area or the amount of peptide or lowering the cell adhesion rate, and also from the viewpoint of preventing shedding by the effects of shear stress when culturing at high density. The term "specific surface area" herein is the surface area per 1 g of dry weight as measured by the BET method.

The cellulose composing the cellulose porous particles described herein were modified with a cationic substituent. The cationic substituent is preferably at least one type selected from the group consisting of primary amino, secondary amino and tertiary amino groups, more preferably primary amino and tertiary amino groups, and even more preferably diethylaminoethyl and 3-amino-2-hydroxypropyl groups. When tertiary amino groups are used for this embodiment, they function as cationic functional groups, and when primary amino groups are used, they function as both cationic functional groups and as condensation sites with the spacer. Using low molecular species with low steric hindrance such as diethylaminoethyl groups or 3-amino-2-hydroxypropyl groups as cationic substituents can reduce the amount of cations within the optimal charge range, allowing more uniform modification. Using high molecular species such as polyethyleneimine with high charge density as cationic substituents, on the other hand, can make it difficult to control the amount of cation to be introduced, potentially resulting in growth inhibition for some cell types.

The charge capacity of the cellulose porous particles of the embodiment, unlike particles of polystyrene or gelatin, must also take into account the effects of hydroxyl groups in the cellulose substrate, while hydrogen bonding and the degree of crystallinity inside the molecules also differs significantly compared to particles of polysaccharides, such as dextran particles. The lower limit for the charge capacity of the cellulose porous particles of the embodiment is preferably 0.01 mmol/g or greater, more preferably 0.05 mmol/g or greater, even more preferably 0.5 mmol/g or greater, even more preferably 0.7 mmol/g or greater, even more preferably 0.9 mmol/g or greater, even more preferably 1 mmol/g or greater and most preferably 2 mmol/g or greater. The upper limit for the charge capacity is also preferably 5.0 mmol/g or lower and more preferably 3.0 mmol/g or lower. If the charge capacity is higher than 0.05 mmol/g it will be possible to efficiently absorb the cells from the start, while if it is lower than 5.0 mmol/g there will be less likelihood of cell growth inhibition during the later growth stages. The "charge capacity", for the present purpose, is the average charge capacity measured for at least 3 samples, by adsorbing chlorine onto the amine groups of the porous particles using hydrochloric acid, and then using a sodium sulfate solution to wash off the adsorbed chlorine and titrating it with silver nitrate using potassium chromate as the indicator.

The polypeptide bonds to the cellulose composing the cellulose porous particles of the embodiment. The arrangement of the polypeptides preferably includes peptides as active sites for cell adhesion factors. One example is the amino acid sequence represented by ArgGlyAsp (RGD) from fibronectin. RGD peptide is a common cell adhesion active sequence in most cell adhesion proteins, and it effectively promotes formation of intersubstrate adhesion between microcarriers and cells. Other cell adhesive polypeptide sequences include, but are not limited to, the amino acid sequences of fibronectin-derived PHSRN and KNEED (one-letter amino acid abbreviations), laminin-derived YIGSR and heparin-derived FHRRIKA. The molecular structure of the polypeptide chain for this embodiment is not particularly limited and may be a straightchain structure, a cyclic structure or a branched structure. Two or more peptides may also be combined, preferably with one or more peptides including the amino acid sequence represented by ArgGlyAsp (RGD).

The number of amino acid residues of the polypeptide may be 5 to 1000 residues, and is preferably 5 to 30 residues. Peptide chains with less than 5 residues have lower physiological effects while peptide chains with 30 or more residues undergo complex entanglement, making it difficult for cells to recognize the adhesion factor sequences that are further inside the peptide molecules.

The lower limit for the polypeptide content is preferably 10 nmol/g or greater, more preferably 100 nmol/g or greater, more preferably 1 $\mu$mol/g or greater, more preferably 1.5 $\mu$mol/g or greater, even more preferably 3 $\mu$mol/g or greater and most preferably 10 $\mu$mol/g or greater, per 1 g of cell culture carrier (dry cellulose porous particles). The upper limit for the polypeptide content is preferably 1 mmol/g or lower, more preferably 500 $\mu$mol/g or lower, even more preferably 200 $\mu$mol/g or lower and most preferably 100 $\mu$mol/g or lower, per 1 g of cell culture carrier (dry cellulose porous particles). The lower limit for the content per unit surface area of the culture carrier is preferably 1 pmol/cm$^2$ or greater, more preferably 10 pmol/cm$^2$ or greater, more preferably 100 pmol/cm$^2$ or greater, more preferably 150 pmol/cm$^2$ or greater, more preferably 300 pmol/cm$^2$ or greater and most preferably 1 nmol/cm$^2$. The upper limit for the content per unit surface area of the culture carrier is preferably 100 nmol/cm$^2$ or lower, more preferably 50 nmol/cm$^2$ or lower, more preferably 20 nmol/cm$^2$ or lower and most preferably 10 nmol/cm$^2$ or lower. If the polypeptide content is too low it will not be possible to increase adhesion or extensibility, while if the content is too high it will be difficult for the polypeptide to be properly oriented due to steric hindrance, thus lowering extensibility of the cells. During cell culturing on a microcarrier, stirring is necessary to cause the microcarrier to be suspended, but this creates shear stress on the cells due to shear force. If the amount of polypeptide and the charge capacity are within the aforementioned ranges, the adhesiveness, proliferation and viability of the cells will be satisfactory even under such conditions. The polypeptide content for this embodiment was calculated by using a fluorophotometer to measure the amount of introduction of a fluorescent molecule (RB-PEG-SH, molecular weight 500, Biochempeg) capable of binding to the maleimide group spacer. More specifically, the amount of unreacted fluorescent molecule accumulated in the solution after the reaction was calculated from the fluorescence intensity of the reaction mixture supernatant, and the value obtained by subtracting this from the amount of fluorescent molecule added to the reaction was used as the amount of introduction.

The polypeptide can be introduced by chemical bonding or physical adsorption, but the preferred method is introduction by chemical bonding to cellulose via a spacer. Herein, the term "spacer" refers to a molecule acting as a bridge between the polypeptide having the cell adhesion peptide sequence, and the cellulose porous particles. For this embodiment, the spacer is preferably a hydrophilic spacer containing an oxygen atom or nitrogen atom, and it is most preferably 3-maleimidepropionic acid. With a simple alkyl linker the surface hydrophobicity increases, lowering the cellulose hydrophilicity and reducing affinity with the cell culture solution. A linker with high hydrophilicity such as PEG, on the other hand, will inhibit adsorption of proteins required for cell adhesion, thus reducing cell adhesion.

The spacer preferably has a portion with rotational freedom and a sterically controlled rigid portion. More preferably it is a molecule having an alkyl group and a cyclic skeleton region, and is most preferably 3-maleimidepropionic acid. By having a cyclic skeleton with high steric hindrance and an alkyl group with a high degree of rotational freedom, it becomes possible to orient the peptide chains in spaces with lower steric hindrance, thus facilitating recognition of the peptide chain by cells.

The chain length of the spacer is not restricted, but the spacer may have a chain length of preferably 5 to 20 atoms and more preferably 5 to 15 atoms. The spacer chain length is the number of atoms forming the spacer when the joint section between the spacer and cellulose porous particles and the joint section between the spacer and the polypeptide have been connected in the shortest possible manner. Examples of constituent atoms include, but are not limited to, carbon atoms, oxygen atoms, nitrogen atoms, sulfur atoms and silicon atoms. If the spacer chain length is less than 5 atoms long the peptide cannot be kept in the proper orientation, thus lowering recognition by cells and reducing adhesion and extensibility of the cells. If the spacer chain length is more than 15 atoms long, on the other hand, introduction efficiency into the porous interiors will be lower due to the chain length, thus likewise reducing the adhesion and extensibility of the cells.

The cellulose porous particles of the embodiment can be produced by a method of molding a solution of cellulose into a desired shape while cooling it to below the solidification temperature for freezing, and then either removing the solvent by extraction or inactivating its dissolution power. The freezing temperature is generally preferred to be set no lower than 40°C below the freezing temperature of the solvent, and will usually be selected in a range of 0 to 20°C lower than the freezing temperature.

The frozen cellulose solution or cellulose derivative solution then has the cellulose- or cellulose derivative-dissolving solvent removed by extraction, or else its solubility is lowered (both of these treatments may also be collectively referred to herein as "solvent removal"), to obtain a solidified cellulose porous body. The conditions for solvent removal are not particularly restricted. It is usually sufficient to rapidly introduce the frozen body into an arbitrary coagulating bath or regenerating bath, with the coagulating bath or regenerating bath preferably being at no higher than the freezing temperature of the solution. A cellulose derivative, however, requires a cellulose regeneration step, where the regeneration is carried out simultaneously with or successively with (i.e. after) the solvent removal. The regeneration itself may be carried out by a common method. Using the production method described above does not require introduction of extraneous materials such as porous materials into the polymer solution during production, and it therefore allows droplets with fine uniform diameters to be easily created, for desired control of the particle sizes. The diameters and shapes of the pores are primarily determined by the sizes and shapes of the solvent crystals formed during freezing solidification of the solvent in the solution. The freezing solidification conditions including the type and temperature of polymer solution may therefore be altered to adjust the shapes and sizes of the pores.

The cellulose porous particles referred to herein may be used directly as granulated cellulose particles, but they are preferably used after crosslinking. The crosslinking method is not restricted, and crosslinking between the cellulose porous body molecules may be accomplished using a crosslinking agent having two or more functional groups that can react and bond with the hydroxyl groups of cellulose. One example is a bifunctional organic substance. An example of such a substance is one of the form X-R-Z [where R represents an aliphatic residue containing a carbon atom, and X and Z are halogens or epoxy groups which are bonded to the carbon atoms of the aliphatic residues], which reacts relatively easily in the presence of an alkaline reactive substance. Examples of bifunctional compounds suitable for reaction include, but are not particularly limited to, epichlorhydrin, dichlorohydrin, 1,2- or 3,4-diepoxybutane, bisepoxypropyl ether, ethylene glycol-bis-epoxypropyl ether, 1,4-butanediol-bis-epoxypropyl ether, and their closely related compounds. Crosslinking allows the crystal structure of the cellulose to be maintained even with abundant modification with cationic substituents, so that the particle structure can be kept stable.

There are no particular restrictions on the method of modifying the cationic substituents on the cellulose composing the cellulose porous particles in the cellulose porous particles of the embodiment, and introduction of groups may be by multiple methods including methods of direct modification of hydroxyl groups and methods of modification via functional groups with epoxy groups. For introduction of a tertiary amino group, a method of directly modifying the hydroxyl groups of the cellulose using 2-(diethylamino)ethyl chloride hydrochloride is preferred. Because of the high abundance of hydroxyl groups on cellulose, it is possible to achieve uniform modification of numerous cationic tertiary amino groups throughout the interiors of the particles by direct modification of hydroxyl groups. For introduction of a primary amino group, the preferred method of modification is reaction with ammonia via a functional group with an epoxy group. Epoxy groups are electrically neutral molecules with low steric hindrance, and they make it possible to uniformly introduce numerous primary amino groups throughout the interiors of the particles even after introduction of the cationic functional group.

The method for introducing the polypeptide into the cellulose composing the cellulose porous particles in production of the cellulose porous particles of the embodiment is not particularly restricted, but it is preferably a method of introduction by chemical bonding, and more preferably a method of reacting a peptide having thiol groups with a spacer having maleimide groups, and bonding the peptide to the cellulose. Specifically, the introduction may be by adding a microcarrier with maleimide groups to an aqueous solution of the polypeptide and stirring. When the peptide has been introduced by physical adsorption there is a risk of shedding of the polypeptide during agitation of the culture, but introduction by chemical bonding can keep the polypeptide on the surface even during agitation. Maleimide groups can react specifically and irreversibly with thiol group-containing peptides, with reaction proceeding under mild conditions in an aqueous solution at pH 6.5 to 7.5, therefore allowing modification to take place without being affected by deformation of the molecular structure or particle structure of the microcarrier. In addition, there is no need to add a condensation agent which may affect culturing, allowing the peptide to be introduced without producing cytotoxicity.

The cellulose porous particles of the embodiment can be used as a culturing microcarrier. The term "culturing microcarrier" here refers to a microcarrier used for adhesion and proliferation of cells to be cultured. In culturing, the desired product may be the cells themselves, or it may be viruses for vaccine production, as a product of cell growth. In the latter case, cells suitable as hosts may be selected. A preferred embodiment may also be using genetically modified cells having a gene introduced as the desired cell growth product. The culturing may be by suspension culture or stationary culture, for example, with no particular limitations on their use.

The cells to be used for culturing are not particularly restricted and may be bacteria, yeast, plant cells or animal cells, including cells from fish or insects, but they are preferably primate or human stem cells. Human stem cells include dental pulp stem cells, neural stem cells, skin stem cells, hematopoietic stem cells, mesenchymal stem cells, mammary stem cells and endothelial stem cells, with no particular limitation to these. Since primate or human stem cells have high scaffolding dependence and strong interaction with cell adhesive peptides, they allow high density culturing to be carried out using a culturing microcarrier comprising cellulose porous particles of the embodiment.

EXAMPLES

The present invention will now be explained in more specific detail through the following Examples and Comparative Examples, with the understanding that the invention is in no way limited to the Examples.

The methods for measuring the physical property values of the structure described above will now be explained.

(1) Charge capacity

A 5.0 g portion of a water-containing sample was measured out and washed twice with 30 mL of aqueous 0.3 N hydrochloric acid and 4 times with 50 mL of aqueous $10^{-4}$ N hydrochloric acid, using a funnel and suction filter. A funnel and suction filter were then used for washing 4 times with 50 mL of a 10% aqueous sodium sulfate solution, and the filtrate was collected. A 0.1 N aqueous potassium chromate solution was added 2 mL each time to the collected filtrate and the mixture was stirred. Titration was performed with a 1 N aqueous silver nitrate solution to determine the titer A (mL).

The sample that had completed washing with the 10% aqueous sodium sulfate solution in the previous step was separately washed 4 times with 50 mL of purified water, and then dried at 105°C for 15 hours or longer, after which the weight was measured with a balance to determine the dry weight B (g).

For measurement of the charge capacity, the measured titer A (mL) and the dry weight B (g) of the sample were used for calculation according to the following formula (1) (N = 3).

$$\text{Charge capacity (mmol/g)} = 1(N)\ (\text{mol/L}) \times A\ (\text{mL})/B\ (\text{g}): \text{Formula (1)}$$

(2) Polypeptide content

The polypeptide content was evaluated using a thiolated fluorescent molecule (RB-PEG-SH, molecular weight: 500, Biochempeg) having similar reactivity with the spacer. A 1.0 g portion of the sample was added to a reaction mixture (purified water: 100 g, WSCD·HCl: 169.8 mg, N-Hydroxysuccinimide (NHS): 282.4 mg, 3-maleimidepropionic acid: 49.4 mg) adjusted to pH 6, and reaction was conducted for 24 hours at 25°C, after which the product was washed. Next, 5 mL of 200 μM thiolated fluorescent molecule was added to 5 mg of the sample, reaction was conducted for 24 hours, and the introduction rate was determined using a fluorophotometer according to the following method. The concentration C ($\mu$mol/L) of the thiolated fluorescent molecule in the supernatant solution after reaction for 24 hours was determined by irradiation of excitation light with a wavelength of 554 nm and measuring the fluorescence intensity at a wavelength of 581 nm, using a fluorophotometer (ARVO X3, PerkinElmer). The amount of RB-PEG-SH introduced into the sample was calculated by the following formula (2) using the concentration of the reacted thiolated fluorescent molecule in the supernatant solution C ($\mu$mol/L) (N = 3).

$$\text{RB-PEG-SH introduction } (\mu\text{mol/g}) = (200 - C) \, (\mu\text{mol/L}) \times 5 \text{ mL/5 mg} \qquad \text{Formula (2)}$$

[Examples 1 to 13, 16 and 17]

A cellulose-copper ammonium solution with a viscosity of 2 poise at 5°C, a cellulose concentration of 2%, a copper concentration of 1.2% and an ammonia concentration of 4%, prepared using refined linter as the starting material, was sprayed in an amount of 500 mL together with nitrogen gas using a two-fluid nozzle, and was used while being stirred in 10 L of silicon oil (SH200,1.5cs, Toray Co., Ltd.) that had been cooled to -40°C. The silicon oil was heated to -20°C while continuing stirring for 20 minutes, and then 10 L of 40% sulfuric acid that had been cooled to -35°C was loaded in. After loading the sulfuric acid, stirring was continued for 8 hours, the temperature was raised to 20°C, and the silicon oil was removed, extracting and washing off the remainder, to obtain porous cellulose particles. A sorting sieve was used to obtain cellulose porous particles having a mean particle size of 240 $\mu$m and a mean pore size of 30 $\mu$m.

Next, 20.0 g of particles were measured out and loaded into a reaction mixture (purified water: 217.30 g, anhydrous sodium sulfate: 88.83 g, sodium dodecylsulfate: 0.016 g, 2-(diethylamino)ethyl chloride hydrochloride aqueous solution (50%): 11.08 g for Examples 1 to 6 and Examples 12 and 13, 0.01 g for Example 7, 2.32 g for Example 8 and Example 16, 22.2 g for Example 9, 44.4 g for Example 10, 220 g for Example 11 and Example 17), and the mixture was stirred. A reaction starting solution (48% aqueous sodium hydroxide: 5.33 g for Examples 1 to 6 and Examples 12 and 13, 0.005 g for Example 7, 1.94 g for Example 8 and Example 16, 10.6 g for Example 9, 21.3 g for Example 10, 110 g for Example 11 and Example 17) was then added and the mixture was gently stirred, after which reaction was conducted while stirring for 1 hour in a thermostatic bath at 70°C. After an elapse of 1 hour, 36% hydrochloric acid was added to pH 7 for neutralization and the sample was washed 3 times by suction filtration with 8000 mL of purified water to obtain cationic cellulose porous particles.

The sample was then combined with a reaction mixture (purified water: 259.74 g, sodium dodecylsulfate: 0.016 g, epichlorhydrin: 37.30 g, 48% aqueous sodium hydroxide solution: 12.65 g) and reaction was conducted while stirring for 1 hour in a thermostatic bath at 60°C. After an elapse of 1 hour, the sample was washed 3 times by suction filtration with 8000 mL of purified water, additional reaction mixture (23% ammonia water solution: 200 g) was added, and reaction was continued while stirring for 1 hour in a thermostatic bath at 40°C. After an elapse of 1 hour, the sample was washed 3 times by suction filtration with 8000 mL of purified water to obtain cationic cellulose porous particles having primary amino groups and tertiary amino groups. Next, 1.0 g of particles was added to a reaction mixture (purified water: 100 g, 1-ethyl-3-(3-dimethylaminopropyl)carbodiimide hydrochloride (WSCD·HCl): 169.8 mg, N-Hydroxysuccinimide (NHS): 282.4 mg, 3-maleimidepropionic acid: 49.4 mg) adjusted to pH 6, and then reacted for 24 hours at 25°C and washed to obtain maleimide group-introduced cellulose porous particles. After then adding a peptide reaction mixture (Example 1: 5 ml aqueous solution of 40 nM CRGDS peptide, Example 2: 5 ml aqueous solution of 200 nM CRGDS peptide, Example 3: 5 ml aqueous solution of 2 $\mu$M CRGDS peptide, Example 4: 5 ml aqueous solution of 20 $\mu$M CRGDS peptide, Example 5: 5 ml aqueous solution of 200 $\mu$M CRGDS peptide, Example 6: 5 ml aqueous solution of 3 mM CRGDS peptide, Examples 7 to 11: 5 ml aqueous solution of 200 $\mu$M CRGDS peptide, Example 12: 5 ml aqueous solution of 200 $\mu$M CGGEGYGEGYIGSR peptide, Example 13: 5 ml aqueous solution of 200 $\mu$M CSTATISGLKPGVDYTITVYAVTGRGD peptide, Example 16: 5 ml aqueous solution of 40 nM CRGDS peptide, Example 17: 5 ml aqueous solution of 40 nM CRGDS peptide) to 78 mg of particles, reaction was conducted for 24 hours at 25°C. After the reaction, the particles were washed with PBS(-) to obtain polypeptide-introduced cellulose porous particles. The charge capacities and polypeptide amounts of the particles are shown in Table 1.

[Example 14]

Cellulose porous particles were obtained by the same method as Example 5, except that glycidyltrimethylammonium chloride was used instead of the aqueous 2-(diethylamino)ethyl chloride hydrochloride (50%) solution.

[Example 15]

A reaction mixture (1.0 g 2-azide-1,3-dimethylimidazolinium hexafluorophosphate, 1.5 ml triethylamine, 250 ml acetonitrile, 250 ml tetrahydrofuran) was added to 6.66 g of cationic cellulose particles prepared by the same method as Example 1, to convert the primary amino groups to azide groups. Next 5 ml of a reaction mixture with a 200 $\mu$M peptide concentration was added to 1.0 g of particles and reaction was conducted for 24 hours at 25°C. The peptide used was a peptide having the G (Propargyl)-RGDS sequence containing a Gly (Propargyl) amino acid residue with an alkyne group on the side chain, and the RGD peptide was introduced by click reaction. After the reaction, the particles were washed with PBS(-) to obtain polypeptide-introduced cellulose porous particles.

The obtained particles were used for 10 days of culturing of hDPSC-dental pulp stem cells (PT-5025, Lonza). A dental pulp stem cell suspension (2.4 mL, 2.25 × 10⁴ cells/mL) was added to 600 $\mu$L of a 10 mg/mL particle suspension obtained by adding the particles to DMEM + 10% FBS medium and cultured by incubation at 37°C. After elapse of a fixed number of days, the particles were sampled and washed 3 times with PBS(-), after which 3 mL of 1 $\mu$M Calcein-AM (Live/Dead Cell Staining Kit II, Protocell) was added and incubation was carried out for 15 minutes while shielded for staining of the cells. After washing 3 times with PBS(-), the particles were irradiated with excitation light at a wavelength of 488 nm using a confocal microscope (FV-3000, Olympus), and a lens with a magnification of 10x was used for observation and photographing of fluorescence at a wavelength of 500 to 540 nm in a depth range of 260 $\mu$m in the Z-axial direction. Analysis software (Image J) was used to calculate the fluorescence area ratio per particle area, and the cell adhesion amount was evaluated. The results are shown in Table 1 below.

Table 1 shows evaluation of adhesion at the start of culturing (viable cell adhesion amount at day 1 of culturing), proliferative ability at the start of culturing (amount of proliferation at day 4 with respect to the viable cell adhesion amount on day 1 of culturing), proliferative ability during culturing (viable cell adhesion amount on day 7 of culturing) and long-term viability (viable cell adhesion amount on day 10 of culturing).

The adhesion at the start (day 1) of culturing was evaluated as VG = ≥150% area ratio, G = ≥130% area ratio, F = ≥100% area ratio or P = <100% area ratio, based on the fluorescence area ratio of Comparative Example 1.

The proliferative ability at the start of culturing was evaluated as VG = ≥400% area increase ratio, G = ≥300% area increase ratio, F = ≥200% area increase ratio or P = <200% area increase ratio, based on the fluorescence area ratio on day 4 with respect to the fluorescence area ratio on day 1 of culturing.

The proliferative ability during culturing was evaluated as VG = ≥800% area increase ratio, G = ≥600% area increase ratio, F = ≥400% area increase ratio or P = <400% area increase ratio, based on the fluorescence area ratio on day 7 with respect to the fluorescence area ratio on day 1 of culturing.

For long-term viability, the fluorescence area ratio on day 10 of culturing was evaluated as VG = ≥400% area ratio, G = ≥200% area ratio, F = ≥150% area ratio or P = <150% area ratio, based on the fluorescence area ratio on day 10 of culturing in Comparative Example 1.

[Comparative Example 1]

Cationic cellulose porous particles were produced by the same method as Example 1 to obtain particles without introduction of maleimide groups or a polypeptide. The charge capacities and polypeptide amounts of the particles are shown in Table 1. The cell proliferation count was also evaluated by the same method as Example 1.

[Comparative Example 2]

Cellulose porous particles were produced by the method of Example 1 by introducing only primary amino groups without introduction of tertiary amino groups, and maleimide groups and a polypeptide were introduced to obtain particles. The charge capacities and polypeptide amounts of the particles are shown in Table 1. The cell proliferation count was also evaluated by the same method as Example 1.

[Table 1]

[Table 1]

| No. | Linker type | Cationic functional group | Peptide type | Peptide amount [$\mu$mol/g] | Charge capacity [mmol/g] | Initial cell adhesion (evaluated on day 1 of culturing) | Initial proliferative ability (evaluated as day 4/day 1 difference) | Proliferative ability (cell count on day 7 of culturing) | Long-term viability (evaluated as cell count on day 10 of culturing) |
|---|---|---|---|---|---|---|---|---|---|
| Example 1 | Maleimide | DEAE | RGD | 0.01 | 1.8 | F | F | F | F |
| Example 2 | Maleimide | DEAE | RGD | 0.059 | 1.8 | G | G | F | G |
| Example 3 | Maleimide | DEAE | RGD | 0.39 | 1.8 | G | G | VG | G |
| Example 4 | Maleimide | DEAE | RGD | 3.2 | 1.8 | VG | VG | VG | G |
| Example 5 | Maleimide | DEAE | RGD | 67 | 1.8 | G | G | G | G |
| Example 6 | Maleimide | DEAE | RGD | 998 | 1.8 | G | G | G | F |
| Example 7 | Maleimide | DEAE | RGD | 67 | 0.05 | F | F | F | F |
| Example 8 | Maleimide | DEAE | RGD | 67 | 0.51 | G | F | G | G |
| Example 9 | Maleimide | DEAE | RGD | 67 | 2.17 | VG | VG | VG | G |
| Example 10 | Maleimide | DEAE | RGD | 67 | 2.95 | VG | VG | VG | VG |
| Example 11 | Maleimide | DEAE | RGD | 67 | 4.95 | VG | VG | VG | G |
| Example 12 | Maleimide | DEAE | YIGSR (CGGEGYGEGYIGSR) | 67 | 1.8 | G | VG | VG | VG |
| Example 13 | Maleimide | DEAE | RGD (CSTATISGLKPGVDYTITVYAVTGRGDS) | 67 | 1.8 | G | VG | VG | VG |
| Example 14 | Maleimide | Quaternary ammonium group | RGD | 67 | 1.8 | VG | VG | VG | VG |
| Example 15 | Azide group | DEAE | RGD | 67 | 1.8 | G | VG | VG | VG |
| Example 16 | Maleimide | DEAE | RGD | 0.01 | 0.51 | F | F | F | F |
| Example 17 | Maleimide | DEAE | RGD | 0.01 | 4.95 | G | G | G | G |

| No. | Linker type | Cationic functional group | Peptide type | Peptide amount [$\mu$mol/g] | Charge capacity [mmol/g] | Initial cell adhesion (evaluated on day 1 of culturing) | Initial proliferative ability (evaluated as day 4/day 1 difference) | Proliferative ability (cell count on day 7 of culturing) | Long-term viability (evaluated as cell count on day 10 of culturing) |
|---|---|---|---|---|---|---|---|---|---|
| Comp. Example 1 | Maleimide | DEAE | RGD | 0 | 1.8 | - | F | F | - |
| Comp. Example 2 | Maleimide | DEAE | RGD | 67 | 0 | P | P | F | P |

INDUSTRIAL APPLICABILITY

The cellulose porous particles of the invention, when used as a culture carrier (culturing microcarrier), allow cells to be efficiently adsorbed and extended during the initial stage of culturing when substrate adhesion between the cells is still undeveloped, while also reducing shedding of cells from the particles by the effects of shear stress even after high density culturing, and allowing the cells to be highly efficiently grown.

**Claims**

1. Cellulose porous particles composed of cellulose, wherein the cellulose composing the cellulose porous particles is modified with a cationic substituent, and the cellulose composing the cellulose porous particles has a polypeptide bonded to the cellulose.

2. The cellulose porous particles according to claim 1, wherein the charge capacity of the porous particles is 0.05 mmol/g to 5.0 mmol/g.

3. The cellulose porous particles according to claim 1 or 2, wherein at least a portion of the polypeptide is a cell adhesive peptide.

4. The cellulose porous particles according to claim 1 or 2, wherein at least a portion of the polypeptide is a peptide having the amino acid sequence ArgGlyAsp (RGD).

5. The cellulose porous particles according to claim 1 or 2, wherein the loading amount of the polypeptide is 10 nmol/g to 1 mmol/g per 1 g of cell culture carrier (dry cellulose porous particles).

6. The cellulose porous particles according to claim 1 or 2, wherein the loading amount of the polypeptide is 1.1 $\mu$mol/g to 1 mmol/g per 1 g of cell culture carrier (dry cellulose porous particles).

7. The cellulose porous particles according to claim 1 or 2, wherein the number of amino acid residues in the polypeptide is 5 to 30 residues.

8. The cellulose porous particles according to claim 1 or 2, wherein the specific surface area of the dry cellulose porous particles is 0.3 $m^2$/g to 4.4 $m^2$/g.

9. The cellulose porous particles according to claim 1 or 2, wherein cellulose has multiple pores, with a mean pore size of 5 $\mu$m to 100 $\mu$m.

10. The cellulose porous particles according to claim 9, wherein the pores form a continuous pore structure with adjacent pores mutually communicating by openings in the membranes dividing them.

11. The cellulose porous particles according to claim 1 or 2, wherein the mean particle size is 100 $\mu$m to 1000 $\mu$m.

12. The cellulose porous particles according to claim 1 or 2, wherein the cationic substituent is at least one selected from the group consisting of primary amino, secondary amino and tertiary amino groups.

13. The cellulose porous particles according to claim 12, wherein the cationic substituent is a tertiary amino group.

14. The cellulose porous particles according to claim 1 or 2, wherein the polypeptide is bonded to the cellulose via a spacer, the chain length of the spacer being 5 to 20 atoms.

15. A culturing microcarrier comprising cellulose porous particles according to claim 1 or 2.

## INTERNATIONAL SEARCH REPORT

International application No.

**PCT/JP2022/045340**

### A. CLASSIFICATION OF SUBJECT MATTER

***C07K 17/12***(2006.01)i; ***C08J 9/28***(2006.01)i; ***C12N 5/0789***(2010.01)i
FI: C07K17/12; C12N5/0789; C08J9/28 101; C08J9/28 CEP

According to International Patent Classification (IPC) or to both national classification and IPC

### B. FIELDS SEARCHED

Minimum documentation searched (classification system followed by classification symbols)

C07K1/00-19/00; C08J9/00-9/42; C12N1/00-7/08

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 4-173086 A (SAKAI ENGINEERING CO., LTD.) 19 June 1992 (1992-06-19) | 1-8, 10-15 |
| | claims, p. 2, lower left column, line 11 to p. 4, upper right column, line 16 | |
| Y | claims, p. 2, lower left column, line 11 to p. 4, upper right column, line 16 | 9 |
| Y | JP 4-91142 A (ASAHI CHEM. IND. CO., LTD.) 24 March 1992 (1992-03-24) | 9 |
| | p. 4, upper left column, line 20 to upper right column, line 11 | |
| A | entire text | 1-8, 10-15 |
| A | JP 2021-153441 A (SHOWA DENKO MATERIALS CO., LTD.) 07 October 2021 (2021-10-07) | 1-15 |
| | entire text | |
| A | JP 2001-321157 A (PRESIDENT OF KYOTO UNIVERSITY) 20 November 2001 (2001-11-20) | 1-15 |
| | entire text | |
| A | JP 59-42452 A (SEKISUI CHEMICAL CO., LTD.) 09 March 1984 (1984-03-09) | 1-15 |
| | entire text | |

☑ Further documents are listed in the continuation of Box C.   ☑ See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 February 2023** | **21 February 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**EP 4 446 346 A1**

**INTERNATIONAL SEARCH REPORT**

International application No.

**PCT/JP2022/045340**

**C.    DOCUMENTS CONSIDERED TO BE RELEVANT**

| Category* | Citation of document, with indication, where appropriate, of the relevant passages | Relevant to claim No. |
|---|---|---|
| A | JP 58-53757 A (DENKI KAGAKU KOGYO K.K.) 30 March 1983 (1983-03-30)<br>entire text | 1-15 |

Form PCT/ISA/210 (second sheet) (January 2015)

14

**INTERNATIONAL SEARCH REPORT**
Information on patent family members

International application No.

**PCT/JP2022/045340**

| Patent document cited in search report | | | Publication date (day/month/year) | Patent family member(s) | Publication date (day/month/year) |
|---|---|---|---|---|---|
| JP | 4-173086 | A | 19 June 1992 | (Family: none) | |
| JP | 4-91142 | A | 24 March 1992 | (Family: none) | |
| JP | 2021-153441 | A | 07 October 2021 | (Family: none) | |
| JP | 2001-321157 | A | 20 November 2001 | (Family: none) | |
| JP | 59-42452 | A | 09 March 1984 | (Family: none) | |
| JP | 58-53757 | A | 30 March 1983 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2208331 A

- JP 5252941 A